# EUROPÄISCHE PATENTSCHRIFT

(11) **EP 1 909 721 B1**
(45) Veröffentlichungstag und Bekanntmachung des Hinweises auf die Patenterteilung: **18.01.2012**
(21) Anmeldenummer: 06762442.9
(22) Anmeldetag: 06.07.2006
(51) Int. Cl.: A61F 13/15

(54) **VERFAHREN ZUM HERSTELLEN EINER VIELZAHL VON ELASTISCHEN, KÖRPERFLÜSSIGKEITEN ABSORBIERENDEN WEGWERFINKONTINENZWINDELN**
METHOD FOR THE PRODUCTION OF A PLURALITY OF ELASTIC DISPOSABLE INCONTINENCE DIAPERS ABSORBING BODY FLUIDS
PROCEDE POUR PRODUIRE DES GARNITURES ELASTIQUES JETABLES POUR L'INCONTINENCE, ABSORBANT DES LIQUIDES ORGANIQUES

(30) Priorität: 09.07.2005 DE 102005032221
(43) Veröffentlichungstag der Anmeldung: 16.04.2008
(73) Patentinhaber: Paul Hartmann Aktiengesellschaft, 89522 Heidenheim (DE)
(72) Erfinder: WURSTER, Thomas, 89522 Heidenheim (DE)
(74) Vertreter: Dreiss, Uwe
(86) Internationale Anmeldenummer: PCT/EP2006/006585
(87) Internationale Veröffentlichungsnummer: WO 2007/006474

(56) Entgegenhaltungen:
- EP-A1- 0 048 011
- WO-A-97/46198
- WO-A-03/057116

## Beschreibung

Die Erfindung betrifft ein Verfahren zum Herstellen einer Vielzahl von elastischen, Körperflüssigkeiten absorbierenden wegwerfinkontinenzwindeln in geschlossener Form (Pantform), häufig auch als Windelhosen bezeichnet.

Wegwerfinkontinenzwindeln in geschlossener Form werden bislang in bekannter Weise in Endlosfertigung unter Verwendung von Vliesstoffbahnen dadurch hergestellt, dass die Vliesstoffbahnen mit einem elastifizierenden Material, in der Regel in Form elastischer Fäden, versehen werden. Es werden im Zuge der Endlosfertigung Abschnitte aus den Vliesstoffbahnen herausgestanzt, um die Beinöffnungen des herzustellenden Windelhöschens zu bilden. Die Vliesstoffbahn wird dann auf sich selbst gefaltet, oder es werden Vliesstoffbahnen übereinander zugeführt und zur Bildung der Seitennähte des Windelhöschens abschnittsweise miteinander verbunden. Dies kann vor oder nach einer Vereinzelung von der endlosen Bahn erfolgen. Beispielhaft wird auf EP 0 048 011 B1 und EP 0 405 575 A1 als Stand der Technik hingewiesen.

WO-A-03 057116 offenbart ein Verfahren zum Herstellen einer Vielzahl von elastischen, Körperflüssigkeiten absorbierenden Wegwerfinkontinenzwindeln in geschlossener Form, die folgenden Verfahrensschritte umfasst:
- Herstellen einer in einer Längsrichtung endlosen, durch einen ersten und einen zweiten Längsrand begrenzten elastischen Materialbahn,
- Zuführen der Materialbahn in der Längsrichtung,
- Bereitstellen und Zuführen von Saugkörpern,
- aufeinanderfolgendes Aufbringen der Saugkörper auf die Materialbahn und Fixieren eines jeweiligen Saugkörpers zwischen zwei Öffnungen auf der Materialbahn,
- Falten der Materialbahn um eine in der Längsrichtung liegende Achse auf sich selbst,
- Fixieren der auf sich selbst gefalteten Materialbahn entlang einer Richtung quer zur Längsrichtung,
- Vereinzeln der Wegwerfinkontinenzwindeln durch Trennen der auf sich selbst gefalteten Materialbahn in vorbestimmten Abschnitten quer zur Längsrichtung.

Bei den bekannten Verfahren erweist es sich als nachteilig, dass zur Elastifizierung des Flächenmaterials der Vliesstoffbahn zusätzliche Mittel vorgesehen und mit dem Flachmaterial in geeigneter Weise verbunden werden müssen. Außerdem ist es als nachteilig anzusehen, dass Ausnehmungen für die Beinöffnungen des herzustellenden Windelhöschens ausgestanzt werden müssen. Hiermit ist ein im Zuge der Endlosfertigung problematischer Schnittabfall verbunden, der aus der Herstellungsmaschine abgeführt und entsorgt werden muss. Außerdem stellt dieser Schnittabfall auch einen nicht nutzbaren Materialverbrauch und damit einen nachteiligen Kostenfaktor dar.

DE 80 03 143 U1, DE 30 04 469 C2 und DE 37 40 845 C1 beschreiben gewirkte Netzhöschen und Verfahren zu deren Herstellung.

Der vorliegenden Erfindung liegt die Aufgabe zugrunde, ein auf wirtschaftliche Weise durchführbares Verfahren der eingangs genannten Art anzugeben, bei welchem die vorgenannten Nachteile nicht auftreten.

Diese Aufgabe wird durch ein Verfahren mit den Merkmalen des Anspruchs 1 erfindungsgemäß gelöst.

Für die die äußere Hülle der Wegwerfinkontinenzwindel bildende Materialbahn wird insbesondere ein textiles Material gewählt, dass in Längsrichtung (entspricht Herstellrichtung) elastisch ist. Vorteilhafterweise ist das textile Material auch in,Querrichtung, das heißt quer zur Herstellrichtung elastisch. Besonders vorteilhaft ist ein Material, dessen elastische Eigenschaften, insbesondere in Bezug auf die Kraft, die das Material der Dehnung entgegensetzt, sich in Längsrichtung und Querrichtung unterscheiden. Insbesondere kann die Kraft, die das Material der Dehnung entgegensetzt, in Längsrichtung geringer sein als in Querrichtung.

Wenn im Sinne der Erfindung von einem textilen Verfahren zum Herstellen der elastischen Materialbahn die Rede ist, so wird hierbei ein Verfahren verstanden, bei dem unter Verwendung von Fäden textile Bindungen gebildet werden, d. h. ein Webverfahren, Wirkverfahren, Strickverfahren oder dergleichen Verfahren. Dies hat den Vorteil, dass bei Ausführung dieses textilen Verfahrens Öffnungen in der Materialbahn gebildet werden können, welche die späteren Beinöffnungen des Windelhöschens bilden, und zwar ohne dass dabei Schnittabfall. resultiert, der abgeführt und entsorgt werden müsste und einen nachteiligen Kostenfaktor darstellt.

Da der Saugkörper auf die noch ungefaltete ebene Materialbahn aufgelegt wird, lässt er sich auch verfahrenstechnisch einfach und erwünschtenfalls über seine gesamte Erstreckung gleichmäßig mit der Flachmaterialbahn derart verbinden, dass er im späteren Gebrauch des Hygieneartikels seine bestimmungsgemäße Position nicht verändert, und zwar auch wenn infolge Körperbewegungen des Benutzers Kräfte auf den Saugkörper ausgeübt werden und Verwindungen des Saugkörpers und der mit ihm verbundenen Materialbahn resultieren. Gewünschtenfalls kann die Herstellung eines hinreichend widerstandsfähigen Verbunds zwischen Saugkörper und Materialbahn durch geeignete Verfahrensmaßnahmen, wie z.B. das Andrücken durch Walzenpaare, unterstützt werden. Erst wenn ein hinreichender Verbund zwischen Saugkörper und Materialbahn innerhalb der Herstellungsmaschine erreicht ist, wird die Materialbahn auf sich selbst gefaltet und zur Bildung der Seitennahtbereiche des Windelhöschens auf sich selbst bzw. mit sich selbst fixiert und daran anschließend vereinzelt.

Nach einer Variante des erfindungsgemäßen Verfahrens werden die die Beinöffnungen einer Wegwerfinkontinenzwindel bildenden Öffnungen in der Längsrichtung der Materialbahn aufeinanderfolgend vorgesehen. Es wird also in der Materialbahn eine einzige in Längsrichtung erstreckte Aufeinanderfolge von Öffnungen gebildet. Die herzustellenden Wegwerfinkontinenzwindeln werden also in ihrer späteren Querrichtung in der Herstellungsmaschine gefördert (Querförderung).

Nach einer anderen Variante werden die die Beinöffnungen einer Wegwerfinkontinenzwindel bildenden Öffnungen paarweise quer zur Längsrichtung der Materialbahn nebeneinander vorgesehen. Dies bedeutet, dass bei Draufsicht auf die Materialbahn zwei in der Längsrichtung erstreckte und parallel zueinander verlaufende Reihen von aufeinanderfolgenden Öffnungen gebildet werden, wobei die zu einer betreffenden Wegwerfinkontinenzwindel gehörenden Öffnungen quer zur Längsrichtung nebeneinander angeordnet sind. Die Wegwerfinkontinenzwindeln werden also in ihrer Längsrichtung gefördert (Längsförderung).

Bei der erstgenannten Verfahrensvariante werden folglich die üblicherweise langgestreckten Saugkörper mit ihrer Längserstreckung quer zur Längsrichtung bzw. Produktionsrichtung des Verfahrens auf der Materialbahn angeordnet. - Es erweist sich bei der ersten Verfahrensvariante als vorteilhaft, wenn die Saugkörper zwischen Gruppen von je zwei in Längsrichtung aufeinanderfolgenden Öffnungen angeordnet werden. Auf diese Weise bildet jede Öffnung in der Materialbahn eine Beinöffnung. Wenn hingegen beim Stand der Technik die Beinöffnungen durch Ausstanzungen bei Vliesstoffmaterialien gebildet werden, so wurde der spätere Trennschnitt zur Vereinzelung der Hygieneartikel durch eine jeweilige Ausstanzung hindurchgeführt, so dass eine jeweilige Ausstanzung für die Bildung zweier Beinöffnungen aneinander angrenzender Artikel zuständig war. Hierdurch wurde aber die Höhe oder Erstreckung des Seitennahtabschnitts der Höschenwindel reduziert. Dies ist nun nicht mehr der Fall. Gleichwohl kann es sich auch als wünschenswert erweisen, wenn zwischen allen Öffnungen in der Materialbahn, welche Beinöffnungen bilden, ein Saugkörper angeordnet wird. Bei der Herstellung in Querförderung resultiert dann eine alternierende Reihenfolge von Öffnung und Saugkörper in der Herstellrichtung. Der Trennschnitt zum Vereinzeln der Artikel muss solchenfalls aber durch eine Beinöffnung verlaufen.

Bei der zweiten Verfahrensvariante (Längsförderung) wird der Saugkörper jeweils zwischen zwei quer zur Längsrichtung nebeneinander vorgesehenen Öffnungen positioniert und in dieser Position fixiert.

Die Positionierung und Fixierung der Saugkörper auf der noch endlosen Materialbahn kann bei beiden Verfahrensvarianten unmittelbar im Anschluss an die Fertigung der Materialbahn erfolgen, gleichsam als ein einziges integriertes Herstellverfahren. Denkbar und vorteilhaft ist aber auch die Trennung dieser Verfahrensschritte, insbesondere derart, dass nach Fertigung der mit den Öffnungen versehenen Materialbahn, diese zunächst zwischengelagert, insbesondere zu einer Endlosrolle aufgewickelt wird. In einem nachgeschalteten Prozess kann dann die Materialbahn wiederum endlos zugeführt und die Positionierung der Saugkörper vorgenommen werden.

Für die Herstellung der Saugkörper werden vorteilhafterweise Vliesstoffmaterialien, insbesondere absorbierende Vliesstoffmaterialien (Nonwoven-Materialien) und/oder superabsorbierende Polymermaterialien verwendet. Es erweist sich des Weiteren als vorteilhaft, wenn für die Bereitstellung des Saugkörpers ein Absorptionskern verwendet wird, der von einem flüssigkeitsdurchlässigen Topsheet (Deckblatt) überfangen und von einem flüssigkeitsundurchlässigen Backsheet (Rückenblatt) unterfangen wird, also zwischen Topsheet und Backsheet angeordnet ist. Auf diese weise kann ein optisch ansprechender, weich anfühlender Saugkörper geschaffen werden, bei dem auch lose im Saugkörper enthaltene Materialien, insbesondere superabsorbierende Polymermaterialien, innerhalb des Saugkörpers gehalten werden und nicht nach außen dringen. Außerdem wird durch das flüssigkeitsundurchlässige Backsheet ein Flüssigkeitsabschluss erreicht. Das Backsheet erstreckt sich vorteilhafterweise vollständig um den Saugkörper herum und bildet ein vorzugsweise auch nach oben kragendes Aufnahmebehältnis für den Absorptionskern. In einer weiteren erfindungsgemäßen Ausführungsform werden Topsheet und Backsheet zumindest abschnittsweise außerhalb des Absorptionskerns miteinander, insbesondere flüssigkeitsdicht verbunden, um eine Aufnahme für den dazwischen angeordneten Absorptionskern zu bilden.

In weiterer Ausbildung der Erfindung erweist es sich als vorteilhaft, dass beidseits des Saugkörpers und in dessen Längsrichtung erstreckt aufstehende Bündchenelemente vorgesehen werden, die einen Seitenauslaufschutz bewirken. Diese Bündchenelemente sind vorzugsweise elastifiziert, insbesondere mit fadenförmigen Elastifizierungsmitteln versehen, welche die Bündchenelemente beim Tragen der Wegwerfinkontinenzwindel gegen den Körper des Benutzers anheben. Es werden so zu den Seiten hin Barrieren für feste und flüssige Körperausscheidungen gebildet.

Wie vorausgehend bereits erwähnt, erweist es sich als vorteilhaft, dass im Zuge der Ausführung des erfindungsgemäßen Verfahrens der Saugkörper hinreichend sicher auf der Materialbahn befestigt werden kann. Nach einer Variante der Erfindung wird der Saugkörper mit seiner gesamten Auflagefläche mit der Materialbahn verbunden. Die Verbindung kann thermisch über ein kontinuierliches oder diskontinuierliches Fügemuster erfolgen. Insbesondere kann die Verbindung über ein Klebemittel, insbesondere ein Heißschmelzklebemittel erfolgen. Das Heißschmelzklebemittel kann wiederum vollflächig oder vorzugsweise in Form eines offenen Musters, beispielsweise als Raster oder sich überlappender Spiralen angeordnet sein.

Nach einer anderen Verfahrensvariante wird der Saugkörper nur mit einer Fläche von höchstens 95 %, insbesondere von höchstens 90 % und weiter insbesondere von höchstens 80 % auf der Materialbahn fixiert. Wiederum werden vorteilhafterweise die zuvor genannten Fügeverfahren und Fügemuster verwendet. Es erweist sich nach dieser Verfahrensvariante als besonders vorteilhaft, wenn lediglich ein zentraler Bereich der körperabgewandten Seite des Saugkörpers auf der Materialbahn fixiert wird. In einer besonders vorteilhaften Ausführungsform bleiben die seitlichen Längsrandbereiche und/oder die Querrandbereiche des Saugkörpers unfixiert. Dies hat bei der Verwendung eines Klebemittelfügeverfahrens den Vorteil, dass ein über die Ränder des Saugkörpers hinausgehender ungenutzter und bei der weiteren Verarbeitung störender Klebemittelauftrag sicher vermieden werden kann. Die unfixierten Ränder des Saugkörpers besitzen eine Erstreckung, gemessen als kürzeste Entfernung vom Saugkörperrand zum Beginn der Fixierung, von insbesondere mindestens 3 mm, weiter insbesondere von mindestens 8 mm, weiter insbesondere von mindestens 15 mm.

Bei der Herstellung des Saugkörpers werden vorteilhafter Weise wenigstens 30 Gew.-%, insbesondere höchstens 90 Gew.-%, insbesondere höchstens 80 Gew.-% und weiter insbesondere höchstens 70 Gew.-% superabsorbierende Polymermaterialien . (SAP) bezogen auf die Masse des Absorptionskerns verwendet.

Es kann sich als vorteilhaft erweisen, dass die in der Längsrichtung liegende Achse, um welche die Materialbahn auf sich selbst gefaltet wird, entlang einer Längsmittellinie der Materialbahn verläuft, also denselben Abstand vom ersten und zweiten Längsrand aufweist.

Obschon für die Auswahl der Materialien zur Herstellung der textilen Materialbahn keine grundsätzliche Beschränkung besteht, erweist es sich als vorteilhaft, wenn hierfür Baumwolle verwendet wird, also Fäden aus oder zumindest mit Baumwolle. Es wäre aber auch denkbar und für bestimmte Anwendungen vorteilhaft, wenn bei der Herstellung der Materialbahn ein thermoplastisches Material verwendet wird, also Fäden aus oder mit einem thermoplastischen Material. Solchenfalls ist es nämlich möglich, dass sich durch Anwendung von Wärme und/oder Druck eine Fügeverbindung herstellen lässt.

Als ganz besonders vorteilhaft erweist es sich, wenn das Fixieren der auf sich selbst gefalteten Materialbahn entlang einer Richtung quer zur Längsrichtung durch thermisches Schweißen oder Ultraschallschweißen durchgeführt wird.

Beim Fixieren der auf sich selbst gefalteten Materialbahn, also beim Ausbilden der in der Regel Seitennahtbereiche bildenden Fügestellen des Windelhöschens, erweist es sich als vorteilhaft, wenn hierfür ein haftvermittelndes Bindemittel verwendet wird. Hierbei kann es sich beispielsweise um ein wärmeschmelzbares thermoplastisches Material handeln oder um einen Kleber, insbesondere einen Heißschmelzkleber, oder um einen streifen- oder fadenförmigen Abschnitt, der eine Haftvermittlung zwischen aufeinander gefalteten Bereichen der Materialbahn auszubilden vermag, insbesondere bei Ausübung von Wärme und/oder Druck.

Nach einer weiteren Variante kann zum Fixieren der auf sich selbst gefalteten Materialbahn entlang einer Richtung quer zur Längsrichtung eine Seitennaht ausgebildet sein, unter Verwendung wenigstens eines weiteren Fadens.

Nach einem weiteren Erfindungsgedanken erweist es sich als vorteilhaft, wenn die auf sich selbst fixierte Materialbahn entlang dieser Fixierung wieder aufreißbar ist. Nach einem noch weiteren Erfindungsgedanken kann es sich als vorteilhaft erweisen, wenn die auf sich selbst fixierte Materialbahn entlang der Fixierung öffenbar und wieder verschließbar ist. Beim Herstellen des Saugkörpers erweist es sich als vorteilhaft, wenn ein atmungsaktives, jedoch gleichwohl flüssigkeitsundurchlässiges Backsheet verwendet wird. Die Atmungsaktivität des Backsheets beträgt solchenfalls wenigstens 300 g/m²/24h , insbesondere wenigstens 1000 g/m²/24h, weiter insbesondere wenigstens 2000 g/m²/24h, weiter insbesondere wenigstens 3000 g/m²/24h, weiter insbesondere wenigstens 4000 g/m²/24h, weiter insbesondere höchstens 6000 g/m²/24h gemessen nach DIN 53 122-1 (Ausgabe: 2001-08).

Dessen ungeachtet erweist es sich als vorteilhaft, wenn beim Herstellen des Saugkörpers ein elastisches Backsheet verwendet wird.

Weiter erweist es sich als vorteilhaft, wenn die elastische Materialbahn mit einer wenigstens doppelt so großen, insbesondere wenigstens dreimal so großen Atmungsaktivität wie die des Backsheets des Saugkörpers ausgebildet wird.

Je nach Faltung der Materialbahn erweist es sich als vorteilhaft, wenn die Wegwerfinkontinenzwindeln nach dem Vereinzeln unter Verwendung einer Wendevorrichtung umgestülpt werden, so dass sie herstellerseitig mit innen liegendem Saugkörper hergestellt und in dieser Form verpackt und an den Letztverbraucher abgegeben werden.

Wenn die Wegwerfinkontinenzwindeln unter Querförderung hergestellt werden, so können die Öffnungen auf einer Längsmittellinie mit gleichem Abstand zu dem ersten und zweiten Längsrand gebildet werden, oder sie können nach einer anderen Variante außermittig, also näher an einem der beiden Längsränder gebildet werden.

Das Zuführen der Materialbahn zum Zeitpunkt des Positionierens des Saugkörpers erfolgt vorteilhafterweise bei einer Bahngeschwindigkeit von wenigstens 50 m/min, insbesondere von wenigstens 100 m/min.

Nach einem weiteren vorteilhaften Erfindungsgedanken wird die Materialbahn in gedehntem Zustand der Herstellungsmaschine zugeführt, und zwar beträgt die Dehnung insbesondere höchstens 20% und weiter insbesondere höchstens 10% ihrer ungedehnten Länge. Dies eröffnet die Möglichkeit, dass der Saugkörper im gedehnten Zustand der Materialbahn auf diese aufgebracht und mit dieser fixiert werden kann, so dass infolge des daran anschließenden elastischen Zusammenziehens der Materialbahn eine Raffung und dadurch eine Elastifizierung des Saugkörpers erhalten wird.

In Weiterbildung dieses Erfindungsgedankens weist die Materialbahn Bereiche unterschiedlicher Elastizität, das heißt insbesondere Bereiche unterschiedlichen E-Moduls auf. Insbesondere ist es denkbar und vorteilhaft, dass die Materialbahn in dem Bereich in dem der Saugkörper zu liegen kommt zumindest abschnittsweise mit einem geringeren E-Modul ausgebildet wird als in dem Bereich, der außerhalb der Saugkörperkontur angeordnet ist.

Schutz beansprucht wird außerdem für eine Wegwerfinkontinenzwindel, die eine textile Außenhülle und einen mit dieser herstellerseitig verbundenen Saugkörper aufweist und durch das erfindungsgemäße Verfahren herstellbar ist.

Nach einer weiteren Ausführungsform des erfindungsgemäßen Verfahrens erweist es sich als vorteilhaft, wenn bei der Ausführung des textilen Verfahrens zwischen zwei die Beinöffnungen bildenden Öffnungen in der Materialbahn eine weitere Öffnung ausgebildet wird, die dann von dem Saugkörper überfangen wird. Auf diese Weise wird also quasi unterhalb des Saugkörpers eine Öffnung in der textilen Außenhülle der herzustellenden Wegwerfinkontinenzwindel gebildet. Dies geht mit einer Materialersparnis einher, hat aber auch weitere Vorteile. Diese Öffnung ist dann quasi durch den Saugkörper abgedeckt, der sich innen vorzugsweise vollumfänglich über die Öffnung hinweg erstreckt. Beispielsweise wäre es auch denkbar, an den verbleibenden verhältnismäßig dünnen Stegen zwischen den Beinöffnungen und dieser zentralen Öffnung eine Materialverstärkung vorzusehen. Es können hier beilspielsweise verstärkende Mittel bei der Ausführung des textilen Verfahrens eingebunden werden. Die Verstärkung kann aber auch einfach durch eine Materialanhäufung der Fäden in diesen Stegbereichen erreicht werden.

Außerdem wird Schutz beansprucht für eine Wegwerfinkontinenzwindel, die im Schrittbereich eine solche Öffnung in der textilen Außenhülle aufweist.

Weitere Merkmale, Einzelheiten und Vorteile der Erfindung ergeben sich aus den beigefügten Patentansprüchen und der zeichnerischen Darstellung und nachfolgenden Beschreibung bevorzugter Ausführungsformen des erfindungsgemäßen Verfahrens. In der Zeichnung zeigt:
- Figur 1: eine schematische Ansicht einer ersten Variante der erfindungsgemäßen Verfahrensführung;
- Figur 2: eine Draufsicht auf die auf sich selbst gefaltete Materialbahn nach der ersten Verfahrensvariante;
- Figur 3: eine schematische Ansicht einer zweiten Variante der erfindungsgemäßen Verfahrensführung;
- Figur 4: eine Draufsicht auf die auf sich selbst gefaltete Materialbahn nach der zweiten Verfahrensvariante;
- Figur 5: eine schematische Draufsicht auf die körperzugewandte Seite einer Windel hergestellt nach der ersten Verfahrensvariante, mit wieder aufgetrennter Seitennaht und aufgefaltet;
- Figur 6: eine Schnittansicht der Windel nach Figur 5;
- Figur 7: eine schematische Ansicht einer weiteren Ausführungsform der ersten Variante der erfindungsgemäßen Verfahrensführung;
- Figur 8: eine Draufsicht auf die auf sich selbst gefaltete Materialbahn nach der Ausführungsform nach Figur 7;
- Figur 9: eine schematische Ansicht einer weiteren Ausführungsform der ersten Variante der erfindungsgemäßen Verfahrensführung und
- Figur 10: eine Draufsicht auf die auf sich selbst gefaltete Materialbahn nach der Ausführungsform nach Figur 9.

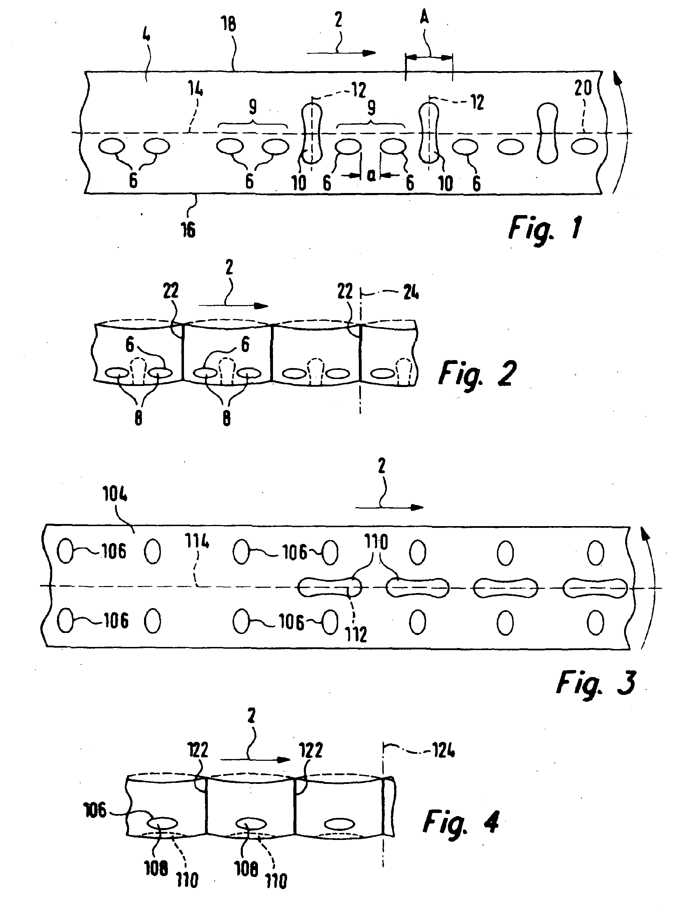

Figur 1 verdeutlicht eine erste Variante eines Verfahrens zum Herstellen einer Vielzahl von elastischen, Körperflüssigkeiten absorbierenden Wegwerfinkontinenzwindeln in geschlossener Form (Pantform), also von Wegwerf-Windelhöschen. Hierfür wird zunächst eine in einer Längsrichtung 2 endlose Materialbahn 4 in einem textilen Verfahren, also z. B. durch weben, Wirken oder Stricken, hergestellt. Im Zuge der Ausführung des textilen Verfahrens, also im Zuge der Herstellung der endlosen, zumindest in Längsrichtung 2 elastischen Materialbahn 4, werden in der Längsrichtung 2 aufeinanderfolgend und in vorgegebenem Abstand voneinander Öffnungen 6 in der Materialbahn gebildet, welche Beinöffnungen 8 der herzustellenden Windelhöschen bilden. Im dargestellten beispielhaften Fall ist der Abstand A zwischen Gruppen 9 von jeweils zwei Öffnungen 6 größer als der Abstand a zwischen den Öffnungen 6 innerhalb einer Gruppe 9.

Die so gebildete Materialbahn 4 wird entweder unmittelbar im Anschluss an ihre endlose Herstellung oder beispielsweise von einer Rolle in der Längsrichtung 2 (Produktionsrichtung) einer Windelherstellungsmaschine zugeführt. Des Weiteren werden Saugkörper 10 bereitgestellt und in der Herstellungsmaschine zugeführt und zwischen die bereits erwähnten Gruppen 9 von jeweils zwei Öffnungen 6 auf die Materialbahn aufgebracht. Der jeweilige Saugkörper 10 wird also im Bereich des größeren Abstands A im hier dargestellten beispielhaften Fall zwischen je zwei Öffnungen 6 auf der Materialbahn 4 angeordnet, und zwar derart, dass seine Längsrichtung 12 quer zur Längsrichtung 2, also der Produktionsrichtung, verläuft. Im dargestellten Fall erkennt man außerdem, dass die Öffnungen 6 außerhalb einer Längsmittellinie 14 der Materialbahn 4 ausgebildet sind, also quer zur Längsrichtung 2 näher an dem einen Längsrand 16 als an dem anderen Längsrand 18 der Materialbahn 4.

Die Saugkörper 10 werden unmittelbar nach dem Aufbringen auf die Materialbahn 4 dauerhaft auf der Materialbahn 4 fixiert, damit sich der Saugkörper 10 beim späteren Gebrauch nicht von der Materialbahn 4 zu lösen vermag. Hierfür können beliebige Fügeverfahren sowie an sich beliebige Haftvermittler, Kleber, Heißschmelzkleber oder dergleichen verwendet werden.

Daran anschließend wird die mit Saugkörpern 10 bestückte Materialbahn 4 um eine in der Längsrichtung liegende Achse 20, die vorliegend mit der Längsmittellinie 14 fluchtet, auf sich selbst gefaltet, so dass die in Figur 2 dargestellte Ansicht resultiert, wobei die Saugkörper 10 innen liegend angeordnet sind. In diesem gefalteten Zustand wird die Materialbahn 4 quer zur Längsrichtung 2 auf sich selbst fixiert, d. h. es wird eine Verbindung der aufeinandergefalteten Abschnitte der Materialbahn 4 quer zur Längsrichtung 2 hergestellt, und zwar in Bereichen zwischen Öffnungen 6, zwischen denen sich kein Saugkörper befindet. Es resultieren quer zur Längsrichtung erstreckte Fügebereiche 22, einer bevorzugten Ausdehnung im Bereich von vorzugsweise mehreren Millimetern, insbesondere 2 bis 12 mm, in der Längsrichtung 2. Die Fügebereiche 22 können vollflächig oder intermittierend, also rasterförmig, ausgebildet sein. Zur Vereinzelung der erfindungsgemäß herzustellenden Windelhöschen wird in den Fügebereichen 22 und entlang der Fügebereiche 22, also quer zur Längsrichtung 2, ein Trennschnitt, der durch die strichpunktierte Linie 24 angedeutet ist, ausgeführt, so dass einzelne Windelhöschen resultieren.

Die in den Figuren 3 und 4 dargestellte weitere Variante des erfindungsgemäßen Verfahrens unterscheidet sich von der in den Figuren 1 und 2 dargestellten Variante dadurch, dass die Windelhöschen unter Längsförderung hergestellt werden. Hierfür wird eine Materialbahn 104 benötigt und hergestellt, bei der Beinöffnungen 106 paarweise quer zur Längsrichtung 2 der Materialbahn 104 nebeneinander in der Materialbahn 104 ausgebildet sind. Es resultieren also in der Materialbahn 104 zwei parallele Reihen von in Längsrichtung 2 aufeinanderfolgenden Öffnungen 106. Zwischen jeweils einem Paar von Öffnungen 106 wird, wie aus Figur 3 ersichtlich, je ein Saugkörper 110 angeordnet, wobei dessen Längsrichtung 112 mit der Längsrichtung 2 und der Längsmittellinie 114 der Materialbahn 104 fluchtet.

Nach dem Fixieren der Saugkörper 110 auf der Materialbahn 104 wird die Materialbahn 104 entlang ihrer Längsmittellinie 114 auf sich selbst gefaltet, so dass die in der Figur 4 dargestellte Anordnung resultiert. Man erkennt in der Draufsicht der Figur 4 jeweils eine Beinöffnung 108 eines herzustellenden Windelhöschens. Wiederum wird die Materialbahn 104 an quer zur Längsrichtung 2 erstreckten Fügebereichen 122 auf sich selbst fixiert, und durch einen Trennschnitt 124 werden die herzustellenden Windelhöschen vereinzeit.

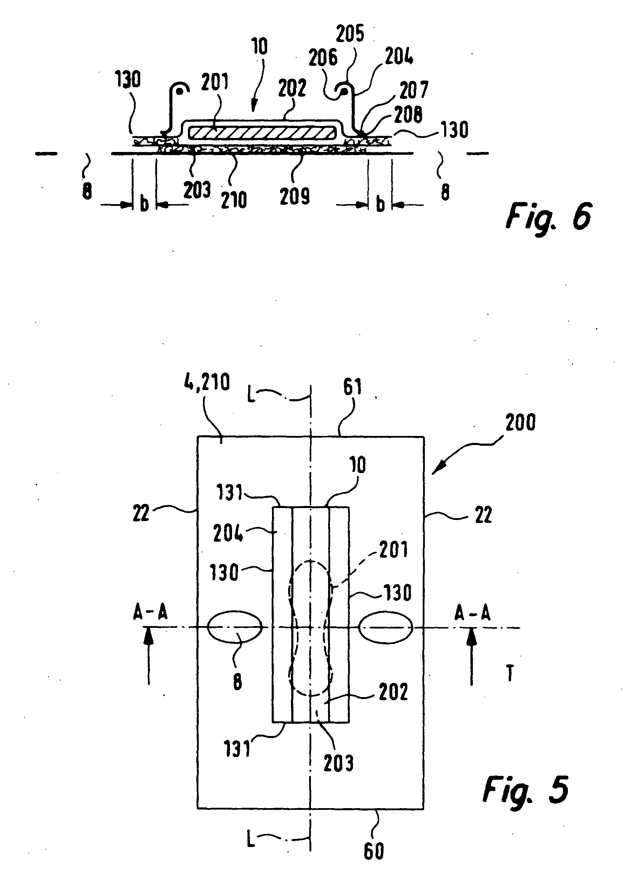

Fig. 5 zeigt schematisch und nicht maßstabsgerecht in der Aufsicht auf die körperzugewandte Seite eine nach der ersten Verfahrensvariante (Figuren 1,2) hergestellte Wegwerfinkontinenzwindel 200, bei der die die Seitennaht der Windel bildenden Fügebereiche 22 aufgetrennt sind und die. Windel aufgefaltet ist. Die Windel weist eine Längsachse L und eine Querachse T auf. Die Querachse T entspricht hierbei der Längsachse 20 (Fig. 1), an der die Materialbahn 4 gefaltet wurde. Erkennbar ist die aus der Materialbahn 4 gebildete äußere, in Richtung der Querachse(T) elastische Hülle 210, die gleichsam das Chassis der Windel bildet, mit einem vorderen Taillenrand 60 und einem hinteren Taillenrand 61 und mit Beinöffnungen 8. Fig. 6 zeigt eine Schnittansicht entlang A-A der Fig. 5. Der Saugkörper 10 weist Längsrandbereiche 130 und Querrandbereiche 131 auf und umfasst einen aus superabsorbierendem Material und Cellulose-Fluff gebildeten Absorptionskern 201, der von einem flüssigkeitsdurchlässigen Topsheet 202 überfangen und einem flüssigkeitsundurchlässigen, jedoch atmungsaktiven Backsheet 203 unterfangen ist. Beidseits des Saugkörpers 10 sind in Längsrichtung erstreckte, zumindest im Bereich der Beinöffnungen (8) aufstehende, Bündchenelemente 204 vorgesehen, an deren distalen Enden 205 Elastifizierungsmittel 206 vorgespannt fixiert sind. Die proximalen Enden 207 der Bündchenelemente sind über ein Heißschmelzklebemittel 208 am Topsheet 202 festgelegt. Topsheet 202 und Backsheet 203 sind außerhalb der Kontur des Absorptionskerns 201 ebenfalls über ein Heißschmelzklebemittel flüssigkeitsdicht verbunden.

Der Saugkörper ist mit etwa 85% der körperabgewandten Seite seines Backsheets 203 mittels eines Heißschmelzklebemittels 209 an der textilen, in Querrichtung elastischen Hülle 210 der Windel 200 festgelegt. Die Fixierung erfolgt hierbei lediglich in einem zentralen Bereich. Die unfixierten äußersten Seiten- 130 und Querrandbereiche 131 des Saugkörpers 10 verbleiben über eine Breite von b = 10 mm unfixiert, das heißt im vorliegenden Fall klebemittelfrei. Die unfixierten Längsränder stehen, hervorgerufen durch die Vorspannwirkung der Elastifizierungsmittel 206 der Bündchenelemente 204 etwas auf (in Figuren 5,6 nicht dargestellt), werden also in Gebrauch in Richtung des Windelträgers angehoben, so dass neben den Bündchenelementen 204 ein weitere seitlicher Auslaufschutzwall gebildet werden kann.

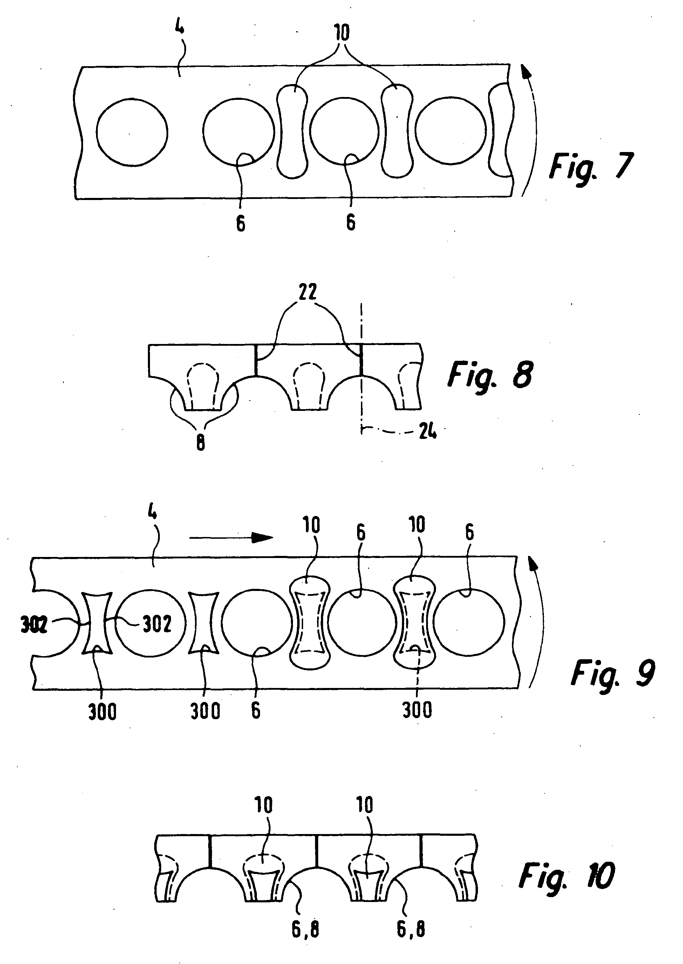

Die Figuren 7 und 8 verdeutlichen eine weitere Ausführungsform der ersten Variante des erfindungsgemäßen Verfahrens, das sich von demjenigen in den Figuren 1 und 2 dargestellten Verfahren dadurch unterscheidet, dass ein jeweiliger Saugkörper 10 nicht zwischen Gruppen von jeweils zwei Öffnungen, sondern alternierend zu den Öffnungen 6 in der Materialbahn 4 auf diese aufgebracht ist. Zwischen allen aufeinanderfolgenden Öffnungen 6, welche Beinöffnungen 8 der herzustellenden Windelhöschen bilden, ist ein Saugkörper 10 angeordnet, was bedeutet, dass beim Vereinzeln im Bereich der Fügebereiche 22 ein jeweiliger Trennschnitt, der durch die strichpunktierte Line 24 in Figur 8 angedeutet ist, durch eine jeweilige Öffnung 6 bzw. 8 verläuft.

Die Figuren 9 und 10 verdeutlichen eine weitere Ausführungsform der ersten Variante des erfindungsgemäßen Verfahrens, bei der wiederum, wie bei Figuren 7 und 8, im Zuge der Herstellung eine alternierende Anordnung von Öffnung 6 und Saugkörper 10 gebildet wird. Es wird jedoch zusätzlich zu den Öffnungen 6, welche Beinöffnungen 8 der herzustellenden Windelhöschen bilden, in der Materialbahn 4 jeweils eine weitere zentrale und im dargestellten Fall unrunde Öffnung 300 im Schrittbereich gebildet. Die Öffnungen 300 werden wie die Öffnungen 6 im Zuge der Ausführung des textilen Verfahrens zur Herstellung der Materialbahn 4 ausgebildet. Im Zuge dessen resultieren im Schrittbereich zwischen den Öffnungen 6 bzw. späteren Beinöffnungen 8 der herzustellenden Windelhose jeweils zwei verhältnismäßig schmale stegförmige Bereiche 302, die einerseits eine Öffnung 6 und andererseits die weitere zentrale Öffnung 300 begrenzen. Es ist durchaus möglich, im Zuge der Herstellung der Materialbahn 4 diese stegförmigen Bereiche 302 gegenüber den übrigen Bereichen der Materialbahn 4 verstärkt auszubilden. Dies ist beispielsweise durch Einarbeiten von Verstärkungsfäden möglich oder aber nur durch eine Konzentration von Fäden bzw. Material. In der gleichen Weise könnten die die Öffnungen 6 begrenzenden Randbereiche der Materialbahn 4 verstärkt ausgebildet werden. Diese Maßnahme ist nicht auf die vorliegende Ausführungsform nach Figuren 9 und 10 beschränkt, sondern könnte auch bei den übrigen dargestellten Verfahrensvarianten verwirklicht werden.

Die so beschriebene weitere zentrale Öffnung 300 wird, wie aus Figur 9 ersichtlich, von einem Saugkörper 10 abgedeckt bzw. von innen überfangen. Der Saugkörper 10 ist dabei so ausgebildet, dass er die Öffnung 300 vorzugsweise vollständig, also allseits, überfängt.

## Patentansprüche

1. Verfahren zum Herstellen einer Vielzahl von elastischen, Körperflüssigkeiten absorbierenden Wegwerfinkontinenzwindeln (200) in geschlossener Form, d.h. in Pantform, die folgenden Verfahrensschritte umfassend:
- Herstellen einer in einer Längsrichtung (2) endlosen, durch einen ersten und einen zweiten Längsrand (16, 18) begrenzten elastischen Materialbahn (4, 104) durch ein textiles Verfahren,
- wobei bei Ausführung des textilen Verfahrens in der Längsrichtung (2) aufeinanderfolgend und in vorgegebenem Abstand voneinander Öffnungen (6, 106) in der Materialbahn (4, 104) gebildet werden,
- Zuführen der Materialbahn (4, 104) in der Längsrichtung (2),
- Bereitstellen und Zuführen von Saugkörpern (10, 110),
- aufeinanderfolgendes Aufbringen der Saugkörper (10, 110) auf die Materialbahn (4, 104) und Fixieren eines jeweiligen Saugkörpers (10, 110) zwischen zwei Öffnungen (6, 106) auf der Materialbahn (4, 104),
- Falten der Materialbahn (4, 104) um eine in der Längsrichtung (2) liegende Achse (20) auf sich selbst,
- Fixieren der auf sich selbst gefalteten Materialbahn (4, 104) entlang einer Richtung (24, 124) quer zur Längsrichtung (2),
- Vereinzeln der Wegwerfinkontinenzwindeln (200) durch Trennen der auf sich selbst gefalteten Materialbahn (4, 104) in vorbestimmten Abschnitten quer zur Längsrichtung (2).

2. Verfahren nach Anspruch 1, **dadurch gekennzeichnet, dass** die die Beinöffnungen (8) einer Wegwerfinkontinenzwindel bildenden Öffnungen (6) in der Längsrichtung (2) der Materialbahn (4) aufeinanderfolgend vorgesehen werden.

3. Verfahren nach Anspruch 1, **dadurch gekennzeichnet, dass** die die Beinöffnungen (108) einer Wegwerfinkontinenzwindel bildenden Öffnungen (106) paarweise quer zur Längsrichtung (2) der Materialbahn (104) nebeneinander vorgesehen werden.

4. Verfahren nach Anspruch 2, **dadurch gekennzeichnet, dass** die Saugkörper (10) zwischen Gruppen (9) von je zwei in Längsrichtung (2) aufeinanderfolgenden Öffnungen (6) angeordnet werden.

5. Verfahren nach Anspruch 2, **dadurch gekennzeichnet, dass** zwischen allen Öffnungen (6) in der Materialbahn (4), welche Beinöffnungen (8) bilden, ein Saugkörper (10) angeordnet wird.

6. Verfahren nach einem oder mehreren der vorstehenden Ansprüche, **dadurch gekennzeichnet, dass** für die Herstellung der Saugkörper (10) Vliesstoffmaterialien
verwendet werden.

7. Verfahren nach einem oder mehreren der vorstehenden Ansprüche, **dadurch gekennzeichnet, dass** für die Bereitstellung des Saugkörpers (10) ein mittig angeordneter Absorptionskern (201) verwendet wird, der von einem flüssigkeitsdurchlässigen Topsheet (202) überfangen und von einem flüssigkeitsundurchlässigen Backsheet (203) unterfangen wird.

8. Verfahren nach einem oder mehreren der vorstehenden Ansprüche, **dadurch gekennzeichnet, dass** beidseits des Saugkörpers (10) und in dessen Längsrichtung (2) erstreckt aufstehende Bündchenelemente (204) vorgesehen werden, die einen Seitenauslaufschutz bewirken.

9. Verfahren nach einem oder mehreren der vorstehenden Ansprüche, **dadurch gekennzeichnet, dass** der Saugkörper (10) nur mit einer Fläche von höchstens 90%, insbesondere von höchstens 80%, insbesondere von höchstens 60% auf der Materialbahn fixiert ist.

10. Verfahren nach einem oder mehreren der vorstehenden Ansprüche, **dadurch gekennzeichnet, dass** der Saugkörper (10) lediglich mit einem zentralen Bereich auf der Materialbahn (4) fixiert ist.

11. Verfahren nach einem oder mehreren der vorstehenden Ansprüche, **dadurch gekennzeichnet, dass** der Saugkörper (10) unter Ausbildung unfixierter freier Längsrandbereiche und/oder unfixierter freier Querrandbereiche auf der Materialbahn (4) fixiert ist

12. Verfahren nach Anspruch 11 **dadurch gekennzeichnet, dass** die unfixierten Längsrandbereiche und/oder Querrandbereiche eine Erstreckung, gemessen als kürzeste Entfernung vom Saugkörperrand zum Beginn der Fixierung, von insbesondere mindestens 3 mm, weiter insbesondere von mindestens 8 mm, weiter insbesondere von mindestens 15 mm aufweisen.

13. Verfahren nach einem oder mehreren der Ansprüche 1-8, **dadurch gekennzeichnet, dass** der Saugkörper (10) vollflächig auf der Materialbahn (4) fixiert wird.

14. Verfahren nach einem oder mehreren der vorstehenden Ansprüche, **dadurch gekennzeichnet, dass** bei der Herstellung des Saugkörpers (10) wenigstens 30 Gew.-%, insbesondere höchstens 90 Gew.-%, insbesondere höchstens 80 Gew.-% und weiter insbesondere höchstens 70 Gew.-% superabsorbierende Polymermaterialien (SAP) bezogen auf die Masse des Absorptionskerns verwendet werden.

15. Verfahren nach einem oder mehreren der vorstehenden Ansprüche, **dadurch gekennzeichnet, dass** die in der Längsrichtung (2) liegende Achse (20), um welche die Materialbahn (4) auf sich selbst gefaltet wird, entlang einer Längsmittellinie (14) verläuft, also denselben Abstand vom ersten und zweiten Längsrand (16 bzw. 18) aufweist.

16. Verfahren nach einem oder mehreren der vorstehenden Ansprüche, **dadurch gekennzeichnet, dass** bei der Herstellung der Materialbahn (4) Baumwolle verwendet wird.

17. Verfahren nach einem oder mehreren der vorstehenden Ansprüche, **dadurch gekennzeichnet, dass** bei der Herstellung der Materialbahn (4) ein thermoplastisches Material verwendet wird.

18. Verfahren nach einem oder mehreren der vorstehenden Ansprüche, **dadurch gekennzeichnet, dass** das Fixieren der auf sich selbst gefalteten Materialbahn (4) entlang einer Richtung quer zur Längsrichtung (2) durch thermisches Schweißen oder Ultraschallschweißen durchgeführt wird.

19. Verfahren nach einem oder mehreren der vorstehenden Ansprüche, **dadurch gekennzeichnet, dass** das Fixieren der auf sich selbst gefalteten Materialbahn (4) entlang einer Richtung quer zur Längsrichtung (2) unter Verwendung eines haftvermittelnden Bindemittels durchgeführt wird.

20. Verfahren nach einem oder mehreren der vorstehenden Ansprüche, **dadurch gekennzeichnet, dass** zum Fixieren der auf sich selbst gefalteten Materialbahn (4) entlang einer Richtung quer zur Längsrichtung (2) eine Seitennaht vorgesehen wird.

21. Verfahren nach einem oder mehreren der vorstehenden Ansprüche, **dadurch gekennzeichnet, dass** die auf sich selbst fixierte Materialbahn (4) entlang der Fixierung wieder aufreißbar ist.

22. Verfahren nach einem oder mehreren der vorstehenden Ansprüche, **dadurch gekennzeichnet, dass** die auf sich selbst fixierte Materialbahn (4) entlang der Fixierung öffenbar und wiederverschließbar ist.

23. Verfahren nach einem oder mehreren der vorstehenden Ansprüche, **dadurch gekennzeichnet, dass** beim Herstellen des Saugkörpers (10) ein atmungsaktives Backsheet (203) verwendet wird.

24. Verfahren nach einem oder mehreren der vorstehenden Ansprüche, **dadurch gekennzeichnet, dass** beim Herstellen des Saugkörpers (10) ein elastisches Backsheet (203) verwendet wird.

25. Verfahren nach einem oder mehreren der vorstehenden Ansprüche, **dadurch gekennzeichnet, dass** die elastische Materialbahn (4) mit einer wenigstens doppelt so großen Atmungsaktivität wie die des Backsheets (203) des Saugkörpers (10) ausgebildet wird.

26. Verfahren nach einem oder mehreren der vorstehenden Ansprüche, **dadurch gekennzeichnet, dass** die Wegwerfinkontinenzwindeln nach dem Vereinzeln unter Verwendung einer Wendevorrichtung umgestülpt werden.

27. Verfahren nach Anspruch 2 und gegebenenfalls einem oder mehreren der Ansprüche 4 bis 26, **dadurch gekennzeichnet, dass** die Öffnungen (6) auf einer Längsmittellinie (14) mit gleichem Abstand zu dem ersten und zweiten Längsrand (16 bzw. 18) gebildet werden.

28. Verfahren nach Anspruch 2 und gegebenenfalls einem oder mehreren der Ansprüche 4 bis 26, **dadurch gekennzeichnet, dass** die Öffnungen (6) außermittig, also näher an einem der beiden Längsränder (16 bzw. 18), gebildet werden.

29. Verfahren nach einem oder mehreren der vorstehenden Ansprüche, **dadurch gekennzeichnet, dass** das Zuführen der Materialbahn (4) während des Aufbringens der Saugkörper (10) bei einer Bahngeschwindigkeit von wenigstens 50 m/min, insbesondere wenigstens 100 m/min erfolgt.

30. Verfahren nach einem oder mehreren der vorstehenden Ansprüche, **dadurch gekennzeichnet, dass** die Materialbahn (4) gedehnt zugeführt wird.

31. Verfahren nach Anspruch 30, **dadurch gekennzeichnet, dass** die Materialbahn (4) um höchstens 20%, insbesondere um höchstens 10% ihrer ungedehnten Länge gedehnt zugeführt wird.

32. Verfahren nach Anspruch 30 oder 31, **dadurch gekennzeichnet, dass** der Saugkörper (10) im gedehnten Zustand der Materialbahn (4) auf diese aufgebracht und mit dieser fixiert wird.

33. Verfahren nach einem oder mehreren der vorstehenden Ansprüche, **dadurch gekennzeichnet, dass** die Materialbahn (4) Bereiche unterschiedlicher Elastizität aufweist.

34. Verfahren nach einem oder mehreren der vorstehenden Ansprüche, **dadurch gekennzeichnet, dass** bei der Ausführung des textilen Verfahrens zwischen zwei die Beinöffnungen (8) bildenden Öffnungen (6) in der Materialbahn (4) eine weitere Öffnung (300) ausgebildet wird, die dann von dem Saugkörper (10) überfangen wird.

35. Körperflüssigkeiten absorbierende Wegwerfinkontinenzwindel (200) in geschlossener Form mit einer textilen elastischen Außenhülle (210) mit Beinöffnungen (8, 108) und einem mit dieser herstellerseitig verbundenen Saugkörper (10, 110), herstellbar durch ein Verfahren nach einem oder mehreren der vorstehenden Ansprüche, in dem die Beinöffnungen (8) im Zuge der Herstellung einer die Außenhülle (210), bildenden Materialbahn (4) in einem textilen Verfahren gebildet sind.

36. Körperflüssigkeiten absorbieren wegwerfinkontinenzwindel (200) nach Anspruch 35, **dadurch gekennzeichnet, dass** zwischen den Beinöffnungen (8) eine weitere zentrale Öffnung (300) in der textilen Außenhülle (210) vorgesehen ist, die von dem Saugkörper (10) von innen überfangen ist.

37. Wegwerfinkontinenzwindel nach Anspruch 36, **dadurch gekennzeichnet, dass** die weitere zentrale Öffnung (300) einer Außenumfangskontur des Saugkörpers (10) entsprechend ausgebildet ist.

38. Wegwerfinkontinenzwindel nach einem der Ansprüche 35 - 37, **dadurch gekennzeichnet, dass** der Saugkörper (10) ein flüssigkeitsdurchlässiges Topsheet (202), ein flüssigkeitsundurchlässiges Backsheet (203) und einen dazwischen angeordneten Absorptionskern (201) umfasst, die eine integrale Einheit bilden und als solche auf die Innenseite der Außenhülle (210) aufgebracht sind.

## Claims

1. A method for the manufacture of a multiplicity of elastic disposable incontinence diapers (200) in closed form, if pants form, for absorbing body fluids, the method comprising the steps of:
- manufacturing, through a textile method, an elastic material web (4, 104) that is continuous in a longitudinal direction (2) and bounded by a first and a second longitudinal edge (16, 18),
- wherein, during carrying out of the textile method, apertures (6, 106) are formed in the material web (4, 104) successively in the longitudinal direction (2) and at a given distance from one another,
- feeding of the material web (4, 104) in the longitudinal direction (2),
- providing and feeding of absorbent bodies (10, 110),
- consecutive application of the absorbent bodies (10, 110) onto the material web (4, 104) and fixing of a respecitve absorbent body (10, 110) between two apertures (6, 106) on the material web (4, 104),
- folding the material web (4, 104) onto itself about an axis (20) located in the longitudinal direction (2),
- fixing the material web (4, 104), folded onto itself, along a direction (24, 124) transverse to the longitudinal direction (2),
- separating the disposable incontinence diapers (200) through severing of the material web (4, 104) folded onto itself in predetermined sections transverse to the longitudinal direction (2).

2. The method according to claim 1, **characterized in that** the apertures (6) forming the leg apertures (8) of a disposable incontinence diaper are provided, one after the other, in the longitudinal direction (2) of the material web (4).

3. The method according to claim 1, **characterized in that** the apertures (106) forming the leg apertures (108) of a disposable incontinence diaper are provided next to each other in pairs transverse to the longitudinal direction (2) of the material web (104).

4. The method according to claim 2, **characterized in that** the absorbent bodies (10) are arranged between successive groups (9) of two apertures (6), one after the other, in the longitudinal direction (2).

5. The method according to claim 2, **characterized in that** an absorbent body (10) is arranged between all apertures (6) in the material web (4) which form leg apertures (8).

6. The method according to any one or several of the preceding claims, **characterized in that** non-woven materials are used for manufacturing the absorbent bodies (10).

7. The method according to any one or several of the preceding claims, **characterized in that** a centrally arranged absorption core (201) is used for the absorbent body (10) which is overlaid by a liquid-permeable top sheet (202) and backed by a liquid-impermeable back sheet (203).

8. The method according to any one or several of the preceding claims, **characterized in that** standing-up cuff elements (204) are provided on both sides of the absorbent body (10) and in the longitudinal direction (2) of said absorbent body, which effect lateral leakage protection.

9. The method according to any one or several of the preceding claims, **characterized in that** the absorbent body (10) is fixed to the material web only with an area of a maximum of 90%, more preferably of a maximum of 80%, more preferably of a maximum of 60%.

10. The method according to any one or several of the preceding claims, **characterized in that** the absorbent body (10) is fixed to the material web (4) only with a central region thereof.

11. The method according to any one or several of the preceding claims, **characterized in that** the absorbent body (10) is fixed to the material web (4) forming unfixed free longitudinal edge regions and/or unfixed free transverse edge regions.

12. The method according to claim 11, **characterized in that** the unfixed longitudinal edge regions and/or transverse edge regions have an extension, measured as shortest distance from the absorbent body edge to the start of the fixation, of preferably at least 3 mm, more preferably of at least 8 mm, more preferably of at least 15 mm.

13. The method according to any one or several of the claims 1-8, **characterized in that** the absorbent body (10) is fixed to the material web (4) with its entire area.

14. The method according to any one or several of the preceding claims, **characterized in that**, during manufacture of the absorbent body (10), at least 30% by weight, especially a maximum of 90% by weight, especially a maximum of 80% by weight, and especially a maximum of 70% by weight of super-absorbent polymer materials (SAP) based on the mass of the absorption core are used.

15. The method according to any one or several of the preceding claims, **characterized in that** the axis (20) disposed in the longitudinal direction (2) about which the material web (4) is folded onto itself is oriented along a longitudinal center line (14), i.e. has the same distance from the first and second longitudinal edges (16 or 18).

16. The method according to any one or several of the preceding claims, **characterized in that** cotton is used in the manufacture of the material web (4).

17. The method according to any one or several of the preceding claims, **characterized in that** a thermoplastic material is used in the manufacture of the material web (4).

18. The method according to any one or several of the preceding claims, **characterized in that** fixing of the material web (4) folded onto itself is carried out along a direction transverse to the longitudinal direction (2) through thermal welding or ultrasonic welding.

19. The method according to any one or several of the preceding claims, **characterized in that** fixing of the material web (4) folded onto itself along a direction transverse to the longitudinal direction (2) is carried out using an adhesion-promoting binder means.

20. The method according to any one or several of the preceding claims, **characterized in that** a lateral seam is provided for fixing of the material web (4) folded onto itself along a direction transverse to the longitudinal direction (2).

21. The method according to any one or several of the preceding claims, **characterized in that** the material web (4), fixed onto itself, is capable of being torn open again along the fixation.

22. The method according to any one or several of the preceding claims, **characterized in that** the material web (4) fixed onto itself is capable of being torn open along the fixation and is closable again.

23. The method according to any one or several of the preceding claims, **characterized in that** a breathable back sheet (203) is used during manufacture of the absorbent body (10).

24. The method according to any one or several of the preceding claims, **characterized in that** an elastic back sheet (203) is used during manufacture of the absorbent body (10).

25. The method according to any one or several of the preceding claims, **characterized in that** the elastic material web (4) is formed with a breathability that is at least twice that of the back sheet (203) of the absorbent body (10).

26. The method according to any one or several of the preceding claims, **characterized in that**, following separation, the disposable incontinence diapers are turned inside out using a reversing device.

27. The method according to claim 2 and if applicable any one or several of the claims 4 to 26, **characterized in that** the apertures (6) are formed on a longitudinal center line (14) with the same distance to the first and second longitudinal edges (16 and 18).

28. The method according to claim 2 and, if applicable, any one or several of the claims 4 to 26, **characterized in that** the apertures (6) are formed off center, i.e. closer to one of the two longitudinal edges (16 or 18).

29. The method according to any one or several of the preceding claims, **characterized in that** the feeding of the material web (4) during application of the absorbent bodies (10) takes place at a web speed of at least 50 m/min, more preferably at least 100 m/min.

30. The method according to any one or several of the preceding claims, **characterized in that** the material web (4) is fed in a stretched state.

31. The method according to claim 30, **characterized in that** the material web (4) is fed stretched by a maximum of 20%, especially by a maximum of 10% of its unstretched length.

32. The method according to claim 30 or 31, **characterized in that** the absorbent body (10) is applied to and fixed with the material web (4) in the stretched state.

33. The method according to any one or several of the preceding claims, **characterized in that** the material web (4) has regions of different elasticity.

34. The method according to any one or several of the preceding claims, **characterized in that**, upon carrying out the textile method, a further aperture (300) is formed in the material web (4) between two apertures (6) forming the leg apertures (8) and is then overlaid by the absorbent body (10).

35. A body fluid absorbing disposable incontinence diaper (200) in closed form with a textile elastic outer skin (210) with leg apertures (8, 108) and an absorbent body (10, 110) joined during manufacturing with said outer skin, the incontinence diaper being manufacturable through a method according to any one or several of the preceding claims, by the leg apertures (8) being formed during the course of manufacture of a material web (4) forming the outer skin (210) in a textile method.

36. The body fluid absorbent disposable incontinence diaper (200) according to claim 34, **characterized in that** a further central aperture (300) is provided in the textile outer skin (210) between the leg apertures (8) and is overlaid from the inside by the absorbent body (10).

37. A disposable incontinence diaper according to claim 36, **characterized in that** the further central aperture (300) is formed corresponding to an outer circumferential contour of the absorbent body (10).

38. The disposable incontinence diaper according to any one of the claims 35 - 37, **characterized in that** the absorbent body (10) comprises a liquid-permeable top sheet (202) a liquid-impermeable back sheet (203) and an absorption core (201) arranged in-between which form an integral unit and are applied as such to the inner side of the outer skin (210).

## Revendications

1. Procédé pour fabriquer plusieurs couches jetables pour personnes incontinentes (200) élastiques absorbant les fluides corporels, se présentant sous forme fermée, c'est-à-dire sous la forme d'une culotte, comprenant les étapes consistant à:
- fabriquer par un procédé textile une bande de matériau (4, 104) élastique sans fin dans le sens longitudinal (2) délimitée par un premier et un deuxième bord longitudinal (16, 18),
- lors de la réalisation du procédé textile, des ouvertures (6, 106) successives dans le sens longitudinal (2) et à distance les unes des autres étant réalisées dans la bande de matériau (4, 104),
- amener la bande de matériau (4, 104) dans le sens longitudinal (2),
- préparer et amener les corps absorbants (10, 110),
- appliquer successivement les corps absorbants (10, 110) sur la bande de matériau (4, 104) et fixer chaque corps absorbant (10, 110) sur la bande de matériau (4, 104) entre les deux ouvertures (6, 106),
- plier la bande de matériau (4, 104) sur elle-même autour d'un axe (20) s'étendant dans le sens longitudinal (2),
- fixer la bande de matériau (4, 104) pliée sur elle-même le long d'une direction (24, 124) transversalement au sens longitudinal (2),
- séparer les couches jetables pour personnes incontinentes (200) en séparant la bande de matériau pliée sur elle-même (4, 104) au niveau de sections prédéfinies transversalement au sens longitudinal (2).

2. Procédé selon la revendication 1, **caractérisé en ce que** les ouvertures (6) formant les ouvertures de jambes (8) d'une couche jetable pour personnes incontinentes sont prévues de façon successive dans le sens longitudinal (2) de la bande de matériau (4).

3. Procédé selon la revendication 1, **caractérisé en ce que** les ouvertures (106) formant les ouvertures de jambes (108) d'une couche jetable pour personnes incontinentes sont prévues deux par deux l'une à côté de l'autre transversalement au sens longitudinal (2) de la bande de matériau (104).

4. Procédé selon la revendication 2, **caractérisé en ce que** les corps absorbants (10) sont agencés entre les groupes (9) de deux ouvertures successives (6) dans le sens longitudinal (2).

5. Procédé selon la revendication 2, **caractérisé en ce qu'**il est agencé un corps absorbant (10) entre toutes les ouvertures (6) réalisées dans la bande de matériau (4) pour former les ouvertures de jambes (8).

6. Procédé selon une ou plusieurs des revendications précédentes, **caractérisé en ce qu'**on utilise pour la fabrication des corps absorbants (10) des matériaux en non-tissé.

7. Procédé selon une ou plusieurs des revendications précédentes, **caractérisé en ce qu'**on utilise pour la préparation du corps absorbant (10) un noyau absorbant (201) agencé au milieu du corps absorbant doublé au-dessus d'une feuille supérieure (202) perméable aux liquides et en-dessous d'une feuille arrière (203) imperméable aux liquides.

8. Procédé selon une ou plusieurs des revendications précédentes, **caractérisé en ce qu'**il est prévu de chaque côté du corps absorbant (10) des éléments de ceinture (204) s'étendant dans le sens longitudinal (2) du corps absorbant, lesquels éléments faisant office de protection latérale contre les fuites.

9. Procédé selon une ou plusieurs des revendications précédentes, **caractérisé en ce que** le corps absorbant (10) est fixé sur la bande de matériau sur une surface au maximum de 90 %, en particulier au maximum de 80 %, en particulier au maximum de 60 %.

10. Procédé selon une ou plusieurs des revendications précédentes, **caractérisé en ce que** le corps absorbant (10) est fixé sur la bande de matériau (4) uniquement sur une zone centrale.

11. Procédé selon une ou plusieurs des revendications précédentes, **caractérisé en ce que** le corps absorbant (10) est fixé sur la bande de matériau (4) par formation de zones de bord longitudinal libres non fixées et/ou de zones de bord transversal libres non fixées.

12. Procédé selon la revendication 11, **caractérisé en ce que** les zones de bord longitudinal et/ou les zones de bord transversal non fixées présentent une étendue, mesurée comme la distance la plus courte entre le bord du corps absorbant et le début de la fixation, en particulier d'au moins 3 mm, plus particulièrement d'au moins 8 mm, plus particulièrement d'au moins 15 mm.

13. Procédé selon une ou plusieurs des revendications 1 à 8, **caractérisé en ce que** le corps absorbant (10) est fixé sur toute la surface de la bande de matériau (4).

14. Procédé selon une ou plusieurs des revendications précédentes, **caractérisé en ce que**, lors de la fabrication du corps absorbant (10), on utilise au moins 30 % en poids, en particulier au maximum 90 % en poids, en particulier au maximum 80 % en poids et plus particulièrement au maximum 70 % en poids de matériaux polymères superabsorbants (SAP) par rapport à la masse du noyau absorbant.

15. Procédé selon une ou plusieurs des revendications précédentes, **caractérisé en ce que** l'axe (20) situé dans le sens longitudinal (2), autour duquel la bande de matériau (4) est pliée sur elle-même, s'étend le long d'une ligne médiane longitudinale (14) et est à égale distance du premier et du deuxième bord longitudinal (16 ou 18).

16. Procédé selon une ou plusieurs des revendications précédentes, **caractérisé en ce que** pour la fabrication de la bande de matériau (4) on utilise du coton.

17. Procédé selon une ou plusieurs des revendications précédentes, **caractérisé en ce que** pour la fabrication de la bande de matériau (4) on utilise un matériau thermoplastique.

18. Procédé selon une ou plusieurs des revendications précédentes, **caractérisé en ce que** la fixation de la bande de matériau (4) pliée sur elle-même le long d'une direction transversale au sens longitudinal (2) est réalisée par soudure thermique ou par soudure à ultrasons.

19. Procédé selon une ou plusieurs des revendications précédentes, **caractérisé en ce que** la fixation de la bande de matériau (4) pliée sur elle-même le long d'une direction transversale au sens longitudinal (2) est réalisée à l'aide d'un liant adhésif.

20. Procédé selon une ou plusieurs des revendications précédentes, **caractérisé en ce que** pour fixer la bande de matériau (4) pliée sur elle-même le long d'une direction transversale au sens longitudinal (2), il est prévu une couture latérale.

21. Procédé selon une ou plusieurs des revendications précédentes, **caractérisé en ce que** la bande de matériau (4) fixée sur elle-même peut être déchirée le long de la fixation.

22. Procédé selon une ou plusieurs des revendications précédentes, **caractérisé en ce que** la bande de matériau (4) fixée sur elle-même peut être ouverte puis refermée le long de la fixation.

23. Procédé selon une ou plusieurs des revendications précédentes, **caractérisé en ce que** pour la fabrication du corps absorbant (10) on utilise une feuille arrière (203) respirante.

24. Procédé selon une ou plusieurs des revendications précédentes, **caractérisé en ce que** pour la fabrication du corps absorbant (10) on utilise une feuille arrière (203) élastique.

25. Procédé selon une ou plusieurs des revendications précédentes, **caractérisé en ce que** la bande de matériau élastique (4) est conçue avec une activité respiratoire au moins deux fois plus grande que celle de la feuille arrière (203) du corps absorbant (10).

26. Procédé selon une ou plusieurs des revendications précédentes, **caractérisé en ce que** les couches jetables pour personnes incontinentes peuvent être retournées, après avoir été séparées, par le biais d'un dispositif de retournement.

27. Procédé selon la revendication 2 et, le cas échéant, selon une ou plusieurs des revendications 4 à 26, **caractérisé en ce que** les ouvertures (6) sont réalisées sur une ligne médiane longitudinale (14) à égale distance du premier et du deuxième bord longitudinal (16 et 18).

28. Procédé selon la revendication 2 et, le cas échéant, selon une ou plusieurs des revendications 4 à 26, **caractérisé en ce que** les ouvertures (6) sont réalisées décentrées, c'est-à-dire plus près de l'un des deux bords longitudinaux (16 ou 18).

29. Procédé selon une ou plusieurs des revendications précédentes, **caractérisé en ce que** l'amenée de la bande de matériau (4) pendant l'application des corps absorbants (10) s'effectue à une vitesse d'avance d'au moins 50 m/min, en particulier d'au moins 100 m/min.

30. Procédé selon une ou plusieurs des revendications précédentes, **caractérisé en ce que** la bande de matériau (4) est amenée étirée.

31. Procédé selon la revendication 30, **caractérisé en ce que** la bande de matériau (4) est amenée étirée au maximum de 20 %, en particulier au maximum de 10 % de sa longueur d'origine.

32. Procédé selon la revendication 30 ou 31, **caractérisé en ce que** le corps absorbant (10) est appliqué et fixé à la bande de matériau (4), à l'état étiré de cette dernière.

33. Procédé selon une ou plusieurs des revendications précédentes, **caractérisé en ce que** la bande de matériau (4) présente des zones d'élasticité différente.

34. Procédé selon une ou plusieurs des revendications précédentes, **caractérisé en ce que** lors de la réalisation du procédé textile, il est réalisé entre deux ouvertures (6) formant les ouvertures de jambes (8) dans la bande de matériau (4) une autre ouverture (300), sur laquelle est ensuite appliqué le corps absorbant (10).

35. Couche jetable pour personnes incontinentes (200) absorbant les liquides corporels, se présentant sous une forme fermée comportant une enveloppe extérieure (210) textile élastique, des ouvertures de jambes (8, 108) et un corps absorbant (10, 110) relié en usine à celle-ci, pouvant être fabriquée par un procédé suivant une ou plusieurs des revendications précédentes, les ouvertures de jambes (8) étant réalisées au cours de la fabrication d'une bande de matériau (4) formant l'enveloppe extérieure (210) dans un procédé textile.

36. Couche jetable pour personnes incontinentes (200) absorbant les liquides corporels selon la revendication 35, **caractérisée en ce qu'**il est prévu entre les ouvertures de jambes (8) une autre ouverture centrale (300) dans l'enveloppe extérieure textile (210), laquelle ouverture est doublée de l'intérieur par le corps absorbant (10).

37. Couche jetable pour personnes incontinentes selon la revendication 36, **caractérisée en ce que** l'autre ouverture centrale (300) est formée par un contour de périphérie extérieur du corps absorbant (10).

38. Couche jetable pour personnes incontinentes selon une des revendications 35 à 37, **caractérisée en ce que** le corps absorbant (10) comprend une feuille supérieure perméable aux liquides (202), une feuille arrière imperméable aux liquides (203) et un noyau absorbant (201) agencé entre les deux, lesquels forment une unité d'un seul tenant et sont appliqués comme tels sur le côté intérieur de l'enveloppe extérieure (210).
